(19) Europäisches Patentamt
European Patent Office
Office européen des brevets

(11) Veröffentlichungsnummer: **0 046 543**
**B1**

# (12) EUROPÄISCHE PATENTSCHRIFT

(45) Veröffentlichungstag der Patentschrift: **02.05.85**

(51) Int. Cl.[4]: **A 61 K 7/06,** A 61 K 7/08, A 61 K 7/13

(21) Anmeldenummer: **81106306.4**

(22) Anmeldetag: **13.08.81**

## (54) Mittel zum gleichzeitigen Färben bzw. Tönen, Waschen und Konditionieren von menschlichen Haaren.

(30) Priorität: **21.08.80 DE 3031535**

(43) Veröffentlichungstag der Anmeldung: **03.03.82 Patentblatt 82/9**

(45) Bekanntmachung des Hinweises auf die Patenterteilung: **02.05.85 Patentblatt 85/18**

(84) Benannte Vertragsstaaten: **AT BE CH DE FR GB IT LI LU NL SE**

(56) Entgegenhaltungen:
**FR - A - 2 190 406**
**FR - A - 2 229 390**
**FR - A - 2 331 325**

**Die Akte enthält technische Angaben, die nach dem Eingang der Anmeldung eingereicht wurden und die nicht in dieser Patentschrift enthalten sind.**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Schrader, Dieter, Itterstrasse 7, D-4000 Düsseldorf 13 (DE)**
Erfinder: **Flemming, Peter, Dr., Rossbachstrasse 45, D-4200 Oberhausen/Sterkrade (DE)**

**EP 0 046 543 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Mittel auf Basis von Oxidationsfarbstoffen zum Färben beziehungsweise Tönen, Waschen und Konditionieren von menschlichen Haaren.

Es ist bekannt, dass das mittels Oxidationsfarbstoffen gefärbte beziehungsweise getönte Haar ohne eine spezielle Nachbehandlung keinen Glanz, einen harten Griff besitzt und nur schlecht kämmbar ist. Um diesem Übelstand abzuhelfen, wurde bereits empfohlen, dem mittels Haarfärbemitteln auf Basis von Oxidationsfarbstoffen gefärbten beziehungsweise getönten und gesondert gewaschenen Haar in einer anschliessenden getrennten Behandlung mit Spülungen auf Basis von kationischen Produkten den vorherigen Zustand von Glanz, Fülle und guter Kämmbarkeit zu verleihen. In der DE-A-2 651 749 wurde bereits vorgeschlagen, diese erforderlichen 3 Schritte der Haarbehandlung Färben/Tönen, Waschen und Konditionieren auf 2 Schritte zu reduzieren. Zu diesem Zweck wurde das Haar zunächst mit einem speziellen Haarfärbemittel, bestehend aus einer Oxidationsfärbekomposition und einem kationischen Polymeren behandelt, und anschliessend mit einem speziellen Shampoo, das wenigstens ein anionisches Tensid und vorzugsweise noch ein kationisches Polymeres enthielt, gewaschen.

Bei den erfindungsgemäss eingesetzten üblichen Oxidationsfarbstoffen handelt es sich um aromatische Verbindungen, die durch Oxidationsmittel zu den eigentlichen färbenden Verbindungen kondensiert werden. Hierbei unterscheidet man zwischen den Entwicklersubstanzen wie zum Beispiel p-Phenylendiamin, p-Toluylendiamin, o-Phenylendiamin, o-Toluylendiamin, p-Aminophenol, p-Aminodiphenylamin, o-Aminophenol, 2,4,5,6-Tetraaminopyrimidin, 4-Aminopyrazolonderivaten, heterocyclischen Hydrazonen und den Kupplerkomponenten wie zum Beispiel Resorcin, 2-Methylresorcin, alpha-Naphthol, 2,7-Dihydroxynaphthalin, Brenzcatechin, Hydrochinon, 1,5-Dihydroxynaphthalin, m-Phenylendiamin und seinen Derivaten, m-Aminophenol, m-Toluylendiamin, 2,4-Diaminoanisol, 2,6-Diaminopyridin.

Die Konzentration der färberischen Zubereitungen an Kuppler-Entwickler-Kombination beträgt 0,02–5 Gewichtsprozent.

Die erfindungsgemäss einzusetzenden üblichen kosmetischen Hilfsmittel dienen in erster Linie dazu, das Mittel in eine verarbeitungsfähige Form wie zum Beispiel Creme, Emulsion, Gel oder auch gegebenenfalls eine einfache Lösung zu bringen. Als solche zusätzlichen Bestandteile sind zum Beispiel Netz- oder Emulgiermittel vom anionischen oder nichtionogenen Typ wie Alkylbenzolsulfonate, Fettsäurealkanolamide, Anlagerungsprodukte von Ethylenoxid an Fettalkohole, Verdickungsmittel wie Methylcellulose, Stärke, höhere Fettalkohole, Paraffinöl, Fettsäuren zu nennen. Diese Zusatzstoffe werden in den für diese Zwecke üblichen Mengen eingesetzt, wie zum Beispiel Netz- und Emulgiermittel in Konzentrationen von 0,5–50 Gewichtsprozent und Verdickungsmittel in Konzentrationen von 0,1–25 Gewichtsprozent, jeweils bezogen auf die gesamte Zubereitung. Darüber hinaus zählen zu den kosmetischen Hilfsmitteln Parfümöle und Haarpflegemittel wie Pantothensäure und Cholesterin.

Geeignete synthetische anionische Tenside sind solche vom Typ der Sulfonate, Sulfate und der synthetischen Carboxylate. Als Tenside vom Sulfonattyp kommen Alkylbenzolsulfonate ($C_{9-15}$-Alkyl), Gemische aus Alken- und Hydroxyalkansulfonaten sowie Disulfonaten, wie man sie beispielsweise aus Monoolefinen mit end- oder innenständiger Doppelbindung durch Sulfonieren mit gasförmigem Schwefeltrioxid und anschliessende alkalische oder saure Hydrolyse der Sulfonierungsprodukte erhält, in Betracht. Weiter eignen sich Alkansulfonate, die aus Alkanen durch Sulfochlorierung oder Sulfoxidation und anschliessende Hydrolyse bzw. Neutralisation bzw. durch Bisulfitaddition an Olefine erhältlich sind. Weitere brauchbare Tenside vom Sulfonattyp sind die Ester von alpha-Sulfofettsäuren, zum Beispiel die alpha-Sulfonsäuren aus hydrierten Methyl- oder Ethylestern, der Kokos-, Palmkern- oder Talgfettsäure.

Geeignete Tenside vom Sulfattyp sind die Schwefelsäuremonoester primärer Alkohole (zum Beispiel aus Kokosfettalkoholen, Talgfettalkoholen oder Oleylalkohol) und diejenigen sekundärer Alkohole. Weiterhin eignen sich sulfatierte Fettsäurealkanolamide, Fettsäuremonoglyceride oder Umsetzungsprodukte von 1–4 Mol Ethylenoxid mit primären oder sekundären Fettalkoholen oder Alkylphenolen.

Weitere geeignete anionische Tenside sind die Fettsäureester beziehungsweise -amide von Hydroxy- oder Amino-carbonsäuren beziehungsweise -sulfonsäuren, wie zum Beispiel die Fettsäuresarcoside, -glykolate, -lactate, -tauride oder -isäthionate.

Besondere Bedeutung unter den anionischen Tensiden kommt dabei den Fettalkoholethersulfaten zu, insbesondere solchen der allgemeinen Formel $R-(CH_2CH_2O)_nCH_2CH_2OSO_3Me$, in der R einen Alkylrest mit 10–16 C-Atomen, n eine Zahl von 1–3 und Me eine salzbildende Base darstellen.

Die anionischen Tenside können in Form ihrer Natrium-, Kalium- und Ammoniumsalze sowie als lösliche Salze organischer Basen, wie Mono-, Di- oder Triethanolamin vorliegen. Sie sind in den erfindungsgemässen Mitteln in einer Menge von 1–25 Gewichtsprozent, bezogen auf das gesamte Mittel, enthalten.

Die erfindungsgemäss einzusetzenden amphoteren oder zwitterionischen Tenside enthalten im Molekül sowohl saure Gruppen, wie zum Beispiel die Carboxyl-, Sulfo-, Schwefelsäurehalbester-, Phosphono- oder Phosphorsäureteilestergruppen, als auch basische Gruppen, wie zum Beispiel Amino-, Imino- oder Ammoniumgruppierungen. Zwitterionische Verbindungen mit einer vierfach substituierten, das heisst quartären Ammoniumgruppe werden als Betaine bezeichnet, wenn sie

im Molekül auch eine kovalent gebundene Säuregruppe besitzen und die positive und negative Ladung intramolekular ausgeglichen ist. Im weiteren Sinne gehören zur Klasse der Betaine auch die entsprechend substituierten quartären Phosphonium- und tertiären Sulfoniumverbindungen. Die oberflächenaktiven Betaine besitzen am Stickstoff im allgemeinen einen höhermolekularen aliphatischen Kohlenwasserstoffrest mit hydrophobem Charakter und zwei niedere Alkylreste mit 1–3 Kohlenstoffatomen, die durch eine oder zwei Hydroxylgruppen substituiert oder direkt oder über ein Heteroatom miteinander verbunden sein können. Die wasserlöslichmachende Carboxylat-, Sulfonat- oder Sulfatgruppe ist über den vierten Substituenten, der meist aus einem kurzkettigen, gegebenenfalls eine Doppelbindung oder eine Hydroxylgruppe aufweisenden aliphatischen Rest besteht, mit dem Ammoniumstickstoff verbunden. Als Tenside sind auch solche Sulfonatbetaine geeignet, bei denen die anionische, wasserlöslichmachende Gruppe direkt an den langkettigen, für die Kapillaraktivität verantwortlichen hydrophoben Kohlenwasserstoffrest gebunden ist. Derartige Sulfobetaine lassen sich beispielsweise aus den aus $C_8$–$C_{20}$-alpha-Olefinen mit $SO_3$ herstellbaren Sultonen und einem tertiären Amin mit drei kurzkettigen aliphatischen Resten erhalten. Typische Vertreter derartiger oberflächenaktiver Betaine sind beispielsweise die Verbindungen 3-(N-Hexadecyl-N,N-dimethyl-ammonium)-propansulfonat; 3-(N-Talgalkyl-N,N-dimethyl-ammonio)-2-hydroxypropansulfonat; 3-(N-Hexadecyl-N,N-bis-(2-hydroxyethyl)-ammonium)-2-hydroxypropylsulfat; 3-(N-Cocosalkyl-N,N-bis(2,3-dihydroxypropyl)-ammonium-propansulfonat; N-Tetradecyl-N,N-dimethyl-ammoniumacetat; N-Hexadecyl-N,N-bis-(2,3-dihydroxypropyl)-ammoniumacetat.

Diese oberflächenaktiven Betaine lassen sich beispielsweise herstellen, indem man das tertiäre Amin mit dem hydrophoben langkettigen aliphatischen Kohlenwasserstoffrest und den beiden niederen Alkyl- oder Hydroxyalkylresten mit Quaternierungsmitteln, wie zum Beispiel 3-Chlor-2-hydroxypropannatriumsulfonat, 3-Chlor-2-hydroxypropylnatriumsulfat, Natriumchloracetat oder Propansulton, Butansulton oder Carbylsulfat umsetzt.

Die amphoteren Tenside sind in den erfindungsgemässen Mitteln in einer Menge von 1–5 Gewichtsprozent, bezogen auf das gesamte Mittel, enthalten. Das Verhältnis von anionischen Tensiden zu amphoteren Tensiden kann sich dabei in den erfindungsgemässen Mitteln in den Bereichen von 5:1 bis 1:1 bewegen.

Bei den in den erfindungsgemässen Mitteln einzusetzenden kationischen Polymeren handelt es sich um Produkte unterschiedlichen Aufbaus, denen die Gegenwart einer Vielzahl quartärer Stickstoffatome gemeinsam ist. Als besonders geeignete Produktgruppe haben sich hierbei die quartären Celluloseetherderivate erwiesen, wie sie in FR-A-1 492 597 beschrieben werden. Die Verbindungen können gemäss dem dort beschriebenen Verfahren durch Verethern und Quaternieren hergestellt werden, wobei die erforderlichen Umsetzungen in beliebiger Reihenfolge oder auch gleichzeitig durchgeführt werden können. Es handelt sich zum Beispiel um Verbindungen, die durch Umsetzung von Hydroxyethylcellulose mit dem Reaktionsprodukt von 0,7 Mol Epichlorhydrin und 0,7 Mol Trimethylamin pro substituierte Anhydroglucoseeinheit gebildet werden. Als unter diese Gruppe der quartären Celluloseetherderivate fallende Handelsprodukte sind die von der Union Carbide vertriebenen Produkte IR 400, IR 30 M und IR 125 zu nennen. Besondere Bedeutung kommt unter den quartären Cellulosederivaten dem Produkt IR 400 zu, welches im Hinblick auf leichte Kämmbarkeit, Glanz und Fülle des behandelten Haares die besten Ergebnisse liefert.

Weitere für den Einsatz in den erfindungsgemässen Mitteln geeignete kationische Polymere stellen Ringstrukturen enthaltende Polymerisate der allgemeinen Formel

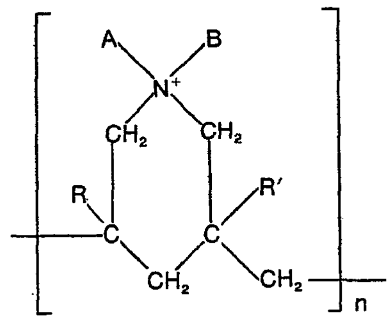

dar, wie sie in der amerikanischen Patentschrift 3 288 770 näher beschrieben sind. Darüber hinaus können grundsätzlich auch alle anderen bekannten quaternierten Polymerisate Verwendung finden, wie sie zum Beispiel in DE-A-2 521 960, DE-A-2 629 922 und US-A-4 175 572 näher beschrieben sind.

Die kationischen Polymeren werden in den erfindungsgemässen Mitteln in einer Menge von 0,5–4 Gewichtsprozent, bezogen auf das gesamte Mittel eingesetzt.

Die Herstellung der erfindungsgemässen Mittel erfolgt in üblicher Weise durch Einarbeiten der Farbstoffkomponenten in eine Trägerbasis in Creme-, Emulsions- oder Gelform, die aus einem Gemisch von Aniontensid, amphoterem Tensid, kationischem Polymeren und weiterhin erforderlichen Netz-, Emulgier-, Verdickungsmitteln, Haarpflegemitteln, Riechstoffen, Wasser besteht. Falls die Erzielung eines gewünschten Farbtones die Mitverwendung direktziehender Farbstoffe erforderlich macht, werden diese gleichfalls mit den Oxidationsfarbstoffkomponenten in die Trägerbasis eingearbeitet.

Die oxidative Kupplung, das heisst die Entwicklung der Färbung, wird zweckmässigerweise mittels chemischer Oxidationsmittel vorgenommen. Als solche kommen insbesondere Wasserstoffperoxid oder dessen Anlagerungsprodukte an Harnstoff, Melamin und Natriumborat sowie Gemische aus derartigen Wasserstoffperoxidanlagerungsverbindungen mit Kaliumperoxiddisulfat in Betracht.

Für die Behandlung des Haares zum gleichzeitigen Färben/Tönen, Waschen und Konditionieren wird die aus Trägerbasis und Farbstoffkomponenten bestehende Zubereitung unmittelbar vor der Anwendung mit einem der genannten Oxidationsmittel vermischt. Die Anwendung der erfindungsgemässen Mittel kann, unabhängig davon, ob es sich um eine Lösung eine Emulsion, eine Creme oder ein Gel handelt, im schwach sauren, neutralen oder insbesondere alkalischen Milieu bei einem pH-Wert von 8–10 erfolgen. Die Anwendungstemperaturen bewegen sich dabei im Bereich von 15 bis 40 °C. Nach einer Einwirkungszeit von 10–45 Minuten wird das Haar mit etwas Wasser eingeschäumt und anschliessend klargespült. Das Haar ist nach dieser einstufigen Behandlung in dem gewünschten Farbton gefärbt beziehungsweise getönt, in feuchtem Zustand leicht kämmbar und nach dem Trocknen glänzend und von gutem Griff. Der Vorteil, der sich durch den Einsatz des erfindungsgemässen Mittels ergibt, besteht darin, dass mit einem einzigen Behandlungsschritt das Haar gefärbt, beziehungsweise getönt, gewaschen, leicht kämmbar gemacht und in den ursprünglichen glänzenden, weichen, griffigen Zustand versetzt werden kann.

Die nachfolgenden Beispiele sollen den Erfindungsgegenstand näher erläutern, ohne ihn jedoch hierauf zu beschränken.

Beispiele

1. Harfärbemittel mittelblond, gleichzeitig waschend und konditionierend

| | |
|---|---|
| Fettalkohol $C_{12-18}$ | 10,5 Gewichtsteile |
| Natriumlaurylethersulfat | 5,0 Gewichtsteile |
| Kokosalkyl-dimethyl-ammoniumbetain | 3,0 Gewichtsteile |
| Polymer IR 400® | 1,0 Gewichtsteile |
| alpha-Naphthol | 0,04 Gewichtsteile |
| 1,3-Bis-(2,4-diamino-phenoxy)-propan | 0,01 Gewichtsteile |
| Resorcin | 0,15 Gewichtsteile |
| p-Toluylendiaminsulfat | 0,21 Gewichtsteile |
| Trilon BS® | 0,20 Gewichtsteile |
| Natriumsulfit | 1,00 Gewichtsteile |
| Ammoniak 25%ig | 6,00 Gewichtsteile |
| Wasser | 72,89 Gewichtsteile |

20 Gewichtsteile vorgenannter Zubereitung wurden unmittelbar vor der Anwendung mit 20 Gewichtsteilen 6%iger Wasserstoffperoxidlösung vermischt. Die Mischung wurde auf hellblondes Haar aufgebracht. Nach 30 Minuten Einwirkungszeit wurde mit wenig Wasser eingeschäumt und anschliessend klargespült. Das Haar war nach der Behandlung mittelblond gefärbt und liess sich in feuchtem Zustand leicht kämmen. Nach dem Trocknen zeigte das Haar einen seidigen Glanz und einen angenehmen, weichen und vollen Griff.

2. Haartönungsmittel kupfergold, gleichzeitig waschend und konditionierend

| | |
|---|---|
| Fettalkohol $C_{12-18}$ | 10,5 Gewichtsteile |
| Natriumkokosalkylethersulfat | 5,0 Gewichtsteile |
| N-Tetradecyl-N,N-dimethyl-ammoniumacetat | 3,0 Gewichtsteile |
| Polymer IR 400® | 1,0 Gewichtsteile |
| 2,4,5,6-Tetraaminopyrimidin-sulfat-Monohydrat | 0,30 Gewichtsteile |
| 2-Methylresorcin | 0,075 Gewichtsteile |
| 2,7-Dihydroxynaphthalin | 0,075 Gewichtsteile |
| m-Aminophenol | 0,01 Gewichtsteile |
| Resorcin | 0,05 Gewichtsteile |
| p-Toluylendiaminsulfat | 0,15 Gewichtsteile |
| Trilon BS® | 0,20 Gewichtsteile |
| Natriumsulfit | 1,00 Gewichtsteile |
| Ammoniak 25%ig | 6,00 Gewichtsteile |
| Wasser | 72,54 Gewichtsteile |

20 Gewichtsteile vorgenannter Zubereitung wurden unmittelbar vor der Anwendung mit 20 Gewichtsteilen 6%iger Wasserstoffperoxidlösung vermischt. Die Mischung wurde auf hellblondes Haar aufgebracht. Nach 20 Minuten Einwirkungszeit wurde mit wenig Wasser eingeschäumt und anschliessend klargespült. Das Haar war nach der Behandlung kupfergold getönt und liess sich in feuchtem Zustand leicht kämmen. Nach dem Trocknen wies das Haar einen seidigen Glanz und einen weichen und vollen Griff auf.

3. Haarfärbemittel schwarz, gleichzeitig waschend und konditionierend

| | |
|---|---|
| Fettalkohol $C_{12-18}$ | 10,5 Gewichtsteile |
| Natriumkokosalkylethersulfat | 5,0 Gewichtsteile |
| N-Hexadecyl-N,N-bis-(2,3-dihydroxypropyl)-ammoniumacetat | 3,0 Gewichtsteile |
| Polymer IR 400® | 1,0 Gewichtsteile |
| 1,3-Bis-(2,4-diamino-phenoxy)-propan | 0,35 Gewichtsteile |
| Resorcin | 1,00 Gewichtsteile |
| p-Toluylendiaminsulfat | 3,00 Gewichtsteile |
| Trilon BS® | 0,20 Gewichtsteile |
| Natriumsulfit | 1,00 Gewichtsteile |
| Ammoniak 25%ig | 8,5 Gewichtsteile |
| Wasser | 66,45 Gewichtsteile |

20 Gewichtsteile vorgenannter Zubereitung wurden unmittelbar vor der Anwendung mit 20 Gewichtsteilen 6%iger Wasserstoffperoxidlösung vermischt. Die Mischung wurde auf mittelblondes Haar aufgebracht. Nach 30 Minuten Einwirkungszeit wurde mit wenig Wasser eingeschäumt und anschliessend klargespült. Das Haar war nach der Behandlung schwarz gefärbt und liess sich in feuchtem Zustand leicht kämmen. Nach dem Trocknen zeigte das Haar einen seidigen Glanz und einen weichen und vollen Griff.

**Patentansprüche**

1. Mittel zum gleichzeitigen Färben bzw. Tönen, Waschen und Konditionieren von menschlichen Haaren, bestehend aus einem Oxidationshaarfärbemittel, das übliche Oxidationsfarbstoffe und kosmetische Hilfsmittel enthält, dadurch gekennzeichnet, dass gleichzeitig

(a) kationische Polymere in einer Menge von 0,5–4,0 Gew.-%

(b) anionische Tenside in einer Menge von 1–25 Gew.-%

(c) amphotere oder zwitterionische Tenside in einer Menge von 1–5 Gew.-%

jeweils bezogen auf das gesamte Mittel, enthalten sind.

2. Mittel nach Anspruch 1, dadurch gekennzeichnet, dass als kationische Polymere quartäre Celluloseetherderivate enthalten sind.

3. Mittel nach Anspruch 2, dadurch gekennzeichnet, dass als kationisches Polymeres das unter der Handelsbezeichnung Polymer JR 400 vertriebene Produkt enthalten ist.

4. Mittel nach Anspruch 1–3, dadurch gekennzeichnet, dass als anionisches Tensid ein Fettalkoholethersulfat, insbesondere ein solches der allgemeinen Formel

$$R-(CH_2-CH_2-O)_n-CH_2-CH_2\ OSO_3\ Me$$

enthalten ist, in der R eine Alkylgruppe mit 10–16 C-Atomen, n eine Zahl von 1–3 und Me eine salzbildende Base darstellen.

5. Mittel nach Anspruch 1–4, dadurch gekennzeichnet, dass das Verhältnis von anionischem Tensid (b) zu amphoterem oder zwitterionischem Tensid (c) 5:1 bis 1:1 beträgt.

6. Mittel nach Anspruch 1–5, dadurch gekennzeichnet, dass sie die Oxidationsfarbstoffe in Gestalt einer Kuppler-Entwickler-Kombination in einer Konzentration von 0,02–5 Gew.-% enthalten.

## Claims

1. Compositions for simultaneously dyeing or tinting, washing and conditioning human hair, consisting of an oxidation hair dye containing standard oxidation dyes and cosmetic auxiliaries, characterized in that they contain

(a) cationic polymers in a quantity of from 0.5 to 4.0% by weight,

(b) anionic surfactants in a quantity of from 1 to 25% by weight and

(c) amphoteric or zwitter-ionic surfactants in a quantity of from 1 to 5% by weight,

based in each case on the composition as a whole.

2. Compositions as claimed in Claim 1, characterized in that they contain quaternary cellulose ether derivatives as the cationic polymer.

3. Compositions as claimed in Claim 2, characterized in that they contain the product marketed as Polymer JR 400 as the cationic polymer.

4. Compositions as claimed in Claims 1 to 3, characterized in that they contain a fatty alcohol ether sulfate, more particularly one of the general formula

$$R-(CH_2-CH_2-O)_n-CH_2-CH_2\ OSO_3\ Me$$

in which R is a $C_{10}$–$C_{16}$ alkyl group, n is a number of 1 to 3 and Me is a salt-forming base as the anionic surfactant.

5. Compositions as claimed in Claims 1 to 4, characterized in that the ratio of anionic surfactant (b) to amphoteric or zwitter-ionic surfactant (c) is 5:1 to 1:1.

6. Compositions as claimed in Claims 1 to 5, characterized in that the contain oxidation dyes in the form of a coupler-developer combination in a concentration of 0.02 to 5% by weight.

## Revendications

1. Agent pour teindre ou nuancer, laver et conditionner simultanément les cheveux humains, consistant en un colorant d'oxydation pour cheveux qui contient les colorants d'oxydation et les auxiliaires cosmétiques usuels, caractérisé en ce que simultanément sont contenus:

(a) des polymères cationiques en une quantité de 0,5 à 4,0% en poids

(b) des tensioactifs anioniques en une quantité de 1 à 25% en poids

(c) des tensioactifs amphotères ou hybrides en une quantité de 1 à 5% en poids,

chaque fois par rapport à l'agent total.

2. Agent selon la revendication 1, caractérisé en ce qu'en tant que polymères cationiques sont contenus des dérivés quaternaires d'éthers de cellulose.

3. Agent selon la revendication 2, caractérisé en ce qu'en tant que polymère cationique est contenu le produit commercialisé sous l'appellation de polymère JR 400.

4. Agent selon les revendications 1 à 3, caractérisé en ce qu'en tant que tensioactif anionique est contenu un sulfate d'éther d'alcool gras, en particulier un de formule générale

$$R-(CH_2-CH_2-O)_n-CH_2-CH_2\ OSO_3\ Me$$

dans laquelle R représente un groupe alcoyle ayant 10 à 16 atomes de carbone, n un nombre de 1 à 3 et Me une base salifiante.

5. Agent selon les revendications 1 à 4, caractérisé en ce que le rapport du tensioactif anionique (b) au tensioactif amphotère ou hybride (c) est de 5:1 à 1:1.

6. Agents selon les revendications 1 à 5, caractérisés en ce qu'ils contiennent les colorants d'oxydation sous la forme d'une combinaison agent coupleur/agent de développement en une concentration de 0,02 à 5% en poids.